# EUROPEAN PATENT APPLICATION

(11) **EP 2 491 996 A1**
(43) Date of publication of application: **29.08.2012**
(21) Application number: 12153465.5
(22) Date of filing: 01.02.2012
(51) Int. Cl.: B01D 46/02, B01D 46/04, B01D 46/42, B01D 46/48, B01D 46/00

(54) **Bag-filter type deodorizing apparatus**

(30) Priority: 27.02.2011 JP 2011058517
(71) Applicant: Nishiyama, Shinroku, Osaka-shi, 532-0012 (JP)
(72) Inventor: Nishiyama, Shinroku, Osaka-shi, 532-0012 (JP)
(74) Representative: De Souza, Paula

(57) **Abstract**

A deodorizing apparatus for deodorizing the ambient air, which is taken through an intake port by a blower, while cyclically supplying powdery activated carbon which serves as a deodorizing powder body to a deodorizing section comprises an auxiliary filtering section having a bag filter section which is fitted to the deodorizing section.

## Description

This invention relates to a bag-filter type deodorizing apparatus for deodorizing the place where odor is emitted like a foundry, a factory for processing odor-generating foods such as fish, or a hospital, for example.

It is required to deodorize the bad-smelling ambient air in the above-mentioned factories, rooms, etc., and to improve the environment.

Generation of odor usually accompanies generation of dust and particulates. The dust and particulates are required to be collected and the odor is required to be removed at the same time. A dust collecting and deodorizing apparatus satisfying both of these requirements has been developed.

Such a dust collecting and deodorizing apparatus as mentioned above is presented by the inventor of this invention in the following documents, for example.
(Patent Document 1) Patent Publication 2009-190019
(Patent Document 2) Patent Publication 2008-30010

In the dust-collecting and deodorizing technology presented in the above-mentioned documents, activated carbon is contained in a filter itself, or quick lime is blown in for a desulfurization purpose. Further, the technology adopts a reflux system in which the activated carbon and the quick lime is thrown into a bag-filter type dust collector, and the activated carbon and the quick lime falling from the bag filter are recovered and thrown into the dust collector again. In this reflux system, the activated carbon etc. are reused and therefore the economic efficiency is improved.

The bag-filter type dust collecting and deodorizing apparatus as mentioned above is a large-scale dust collector for collecting the dust of the entire foundry or the like which essentially places priority on a dust collecting function. When deodorization is performed by this kind of large-scale dust collector, a large quantity of expensive activated carbon etc. is needed. In addition, while the air discharged to the atmosphere outside the factory becomes free from odor, there still remains a problem in a work site.

Namely, odor is emitted locally, and the problem that local workers at the work site are exposed to the odor has not been solved. To be specific, the odor is generated in a foundry for aluminum casting, aluminum die-casting, etc. where a core of a shell-mold or the like is used, in a medical facility like a hospital, in a chemical factory using chemicals, in various kinds of laboratories etc. where abnormal odor is generated, while requirements for ventilation for each case are stipulated by regulations. The odor is locally generated in these places and the local workers at the site are exposed to the odor.

In order to solve the problem, a small-scale deodorizing and dust collecting device which can be installed near a molding machine, for example, is desired

The bag-filter type deodorizing apparatus as mentioned above is structured so that powdery activated carbon can be cyclically used, whereby the bag-filter type deodorizing apparatus has progressed dramatically in a sense that both of the requirements for dust collection and deodorization are satisfied, that the powdery activated carbon is cyclically used, and that the deodorization function is improved.

However, the bag-filter type dust-collecting and deodorizing apparatus has the problem that the powdery activated carbon may be discharged outside if the bag filter should be broken for any reason during the cyclic use of the powdery activated carbon.

Further, even when the deodorizing and dust collecting device is small-scale, the noise caused by intake and discharge of the air by a blower is considerable. To be specific, the noise level in reality is around as much as 80 dB. The noise is not problematic where there are other sources of noise, however, when the apparatus is installed and used where there is less noise like in a hospital, an office, or a food processing factory, there arises a problem.

The purposes of this invention are to prevent the powdery activated carbon from being easily discharged outside even when the bag filter in a deodorizing section is broken, and to substantially reduce the noise during operation, while exerting a continuous deodorizing function by the cyclic use of the powdery activated carbon as well as exerting a dust-collecting function.

To achieve the above-mentioned purposes, the bag-filter type deodorizing apparatus of this invention including a deodorizing section 3 having the structure similar to that of a bag-filter type dust-collecting structure provided with a bag filter section 1 and a pulse jet mechanism 2 dusting off the bag filter section 1, and a cyclical supply section 4 cyclically supplying the powdery activated carbon P serving as a deodorizing powder body to the deodorizing section 3, wherein the ambient air to be deodorized which is taken in through an intake port 5 by a blower 13 for discharging the air is deodorized by using the powdery activated carbon P and is discharged through a discharge port 6, comprises an auxiliary filtering section 7 provided with a bag filter section 10 fitted to the deodorizing section 3. The blower 13 is arranged between the deodorizing section 3 and the auxiliary filtering section 7. The ambient air deodorized by the deodorizing section 3 is sent into the auxiliary filtering section 7, and is discharged outside through the auxiliary filtering section 7.

According to this invention, the ambient air to be deodorized means all the ambient air in the foundry, the food processing factory, the hospital, the laboratory using chemicals, etc. where deodorization is required and the work environment has to be improved.

The deodorizing section of this invention structured in a similar manner to that of the bag-filter type dust collector is publicly known. Namely, the ambient air is taken in by the blower, under a negative pressure, from one side of the bag-shaped filter arranged in a dust collecting chamber having an intake port or an intake duct, and the air is discharged to the outside from the other side of the bag filter. The bag filter is vibrated at predetermined intervals by the pulse jet mechanism to resolve clogging.

In the bag-filter type deodorizing apparatus according to this invention which exerts both functions of continuous deodorization by cyclic use of the powdery activated carbon and of dust collection, the powdery activated carbon is not allowed to be easily discharged to the outside even when the bag filter in the deodorizing section is broken, by additionally arranging an auxiliary filtering section provided with a bag-filter section. At the same time, the ambient air to be deodorized is sent to and is allowed to pass through, by the blower, the auxiliary filtering section provided with the bag filter section, wherefore, the bag-filter section exerts a buffering function, and the noise level which has been 80 dB is suppressed to around 55 dB. This is a conspicuous advantage.

Other specific advantages will be clarified in the following explanation.

In embodying the invention, it is preferable to structure the deodorizing apparatus in a manner that the blower 13 for discharging the air is arranged above the deodorizing section 3, and the auxiliary filtering section 7 is arranged above the deodorizing section 3 and beside the blower 13 so that the ambient air to be deodorized which is taken in from underneath may be sent sideways, be allowed to pass through the bag filter section 10 of the auxiliary filtering section 7, and be discharged to the outside.

Thus, the entire deodorizing apparatus is formed to be compact, which is another advantage.

Further, the bag filter section 1 of the deodorizing section 3 and the bag filter section 10 of the auxiliary filtering section 7 are preferably arranged in a manner that the longitudinal direction of the bag-shaped body of the bag filter is directed sideways.

In a general dust collecting apparatus, the bag filter is conventionally arranged vertically in a suspending manner. This arrangement makes the apparatus higher, and a large installation space is required. Therefore, the apparatus cannot be installed in a room like an ordinary office.

However, according to this invention, the height is suppressed to about 2 m enabling installation in an ordinary room. The air-treatment capacity is 30m³/min in this embodiment.

Further, it is preferable that quick lime in the powdery state is mixed in the powdery activated carbon P, whereby CO₂ in the ambient air to be deodorized is reactively absorbed, and other poisonous gas and fine dust are adsorbed, so that the ambient air to be deodorized is further cleaned.

Further, it is preferable that an antibacterial material AB in the powdery state is mixed into the powdery activated carbon P. Various molds and bacterium contained in the odor of the ambient air to be deodorized are prevented from being discharged outside by passing through two bag filter sections 1 and 10, and the air is discharged in a state of being sterilized to the extent possible.
Fig. 1 is a front view of an entire bag-filter type deodorizing apparatus of this invention.
Fig. 2 is a right side view of the entire bag-filter type deodorizing apparatus.
Fig. 3 is a rear view of the bag-filter type deodorizing apparatus.
Fig. 4 is a plan view of the bag-filter type deodorizing apparatus.
Fig. 5 is a cross sectional view of the bag-filter type deodorizing apparatus taken in the direction of arrows A-A in Fig. 2.
Fig. 6 is a left side view of the entire bag-filter type deodorizing apparatus.
Fig. 7 is a plan view showing piping of the bag-filter type deodorizing apparatus.

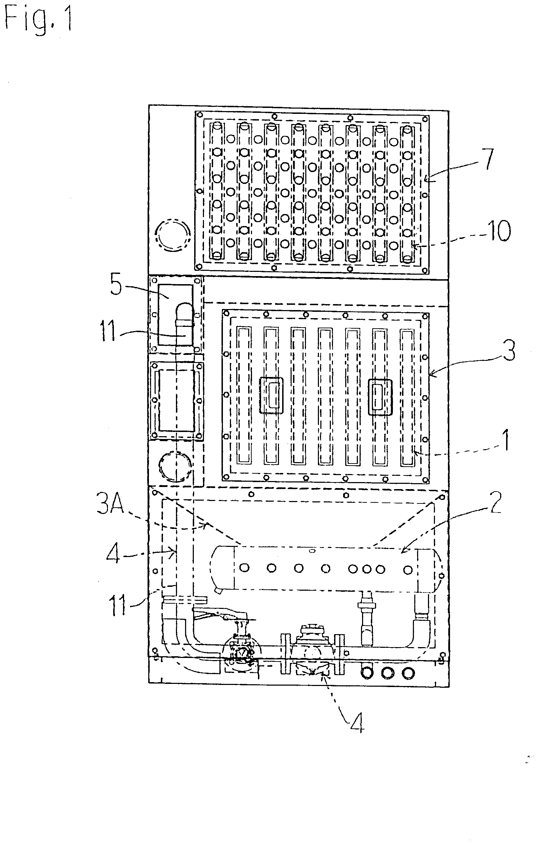

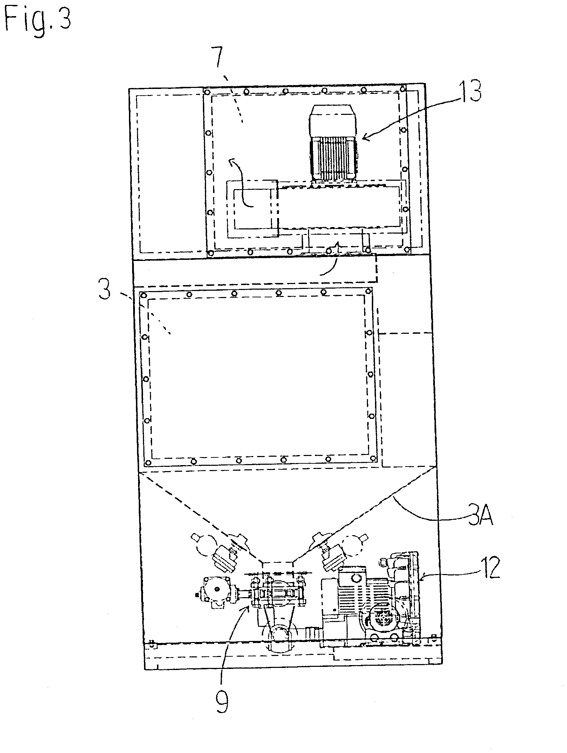

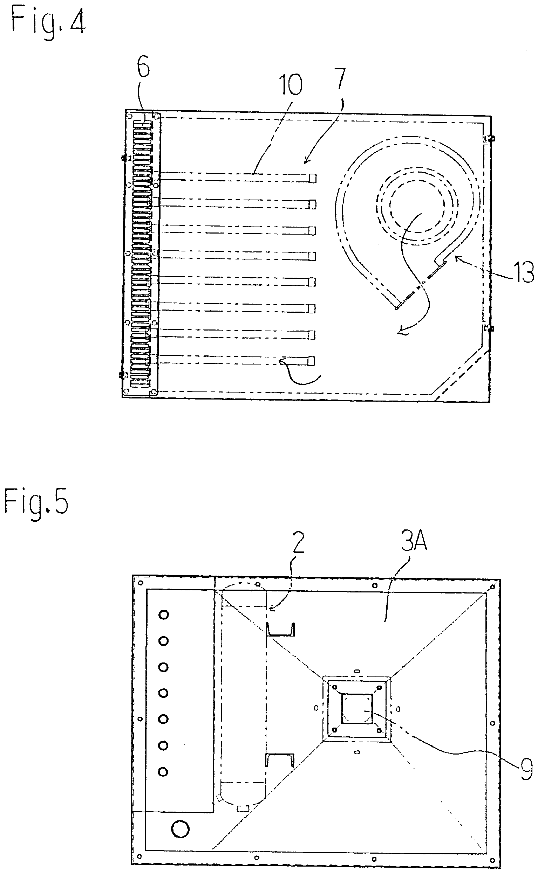

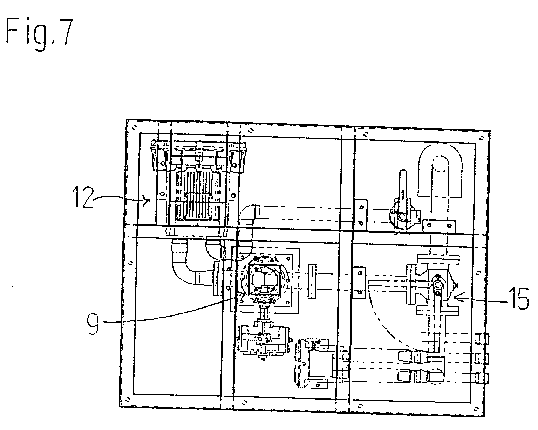

A preferred embodiment of the bag-filter type deodorizing apparatus of this invention will be described in detail hereinafter based on Figs. 1 to 7. In this embodiment, explanation is given taking the bad-smelling air in a food processing factory (a fish processing factory) as the ambient air to be deodorized.

The bag-filter type deodorizing apparatus includes a deodorizing section 3 and a cyclical supply section 4. The deodorizing section 3 has the structure similar to the bag-filter type dust collecting structure provided with a bag filter section 1 (Filter cloth of Tetoron felt: "Tetoron" is a registered trademark.) having a plurality of bag-shaped filter cloths, and a pulse jet mechanism 2 dusting off the bag filter section 1 (Filter cloth of Tetoron felt: "Tetoron" is a registered trademark.). The cyclical supply section 4 cyclically supplies the powdery activated carbon P, serving as the deodorizing powder body, to the deodorizing section 3. The ambient air to be deodorized which is taken through the intake port 5 by the blower 13 for discharging the air is deodorized by using the powdery activated carbon P, and discharged through the discharge port 6.

The fundamental structure as mentioned above is substantially same as that presented by the inventor of this invention in the patent publication 2009-190019 of the cited document 1.

According to the present invention, the auxiliary filtering section 7, provided with a bag filter section 10 is fitted to the deodorizing section 3. The blower 13 is arranged between the deodorizing section 3 and the auxiliary filtering section 7. The ambient air deodorized by the deodorizing section 3 is sent into the auxiliary filtering section 7, and is discharged outside through the auxiliary filtering section 7.

To be specific, the blower 13 is arranged above the deodorizing section 3, and the auxiliary filtering section 7 is arranged above the deodorizing section 3 and beside the blower 13. The deodorized ambient air taken from the lower section is sent sideways and is discharged to the outside through the bag filter section 10 of the auxiliary filtering section 7. The bag filter section 10 is not provided with the pulse jet mechanism 2.

The bag-filter section 1 (7 lateral rows) of the deodorizing section 3 and the bag filter section 10 (8 lateral rows) of the auxiliary filtering section 7 are arranged in a manner that the longitudinal direction of the bag-shaped bodies of the bag filters are oriented in a horizontal direction but not in a vertical direction.

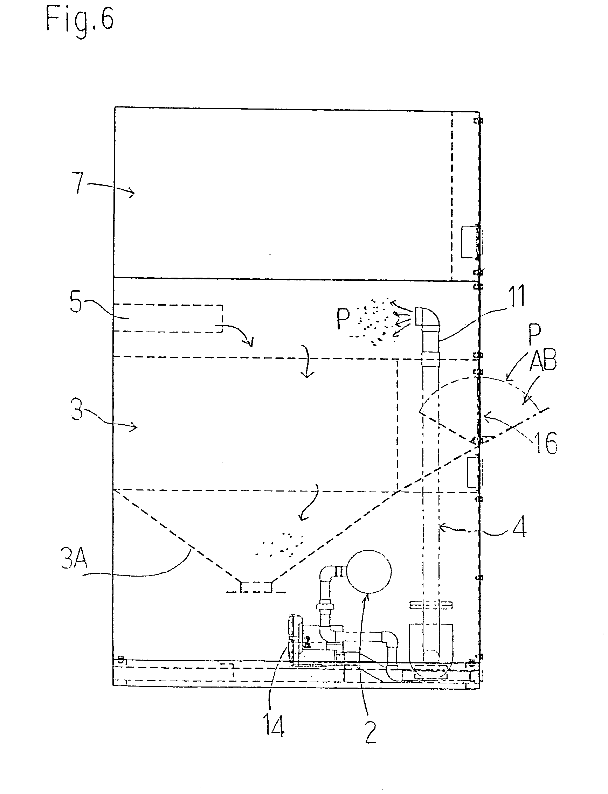

The powdery activated carbon P used in this embodiment is a fine powder having a particle size of around 60 µ. Otherwise, the size may be 200 to 2000 meshes, and the powder body of 1 mm or less may be mixed. In this embodiment, the weight ratio of the quick lime in the powder state to the powdery activated carbon P is 1 to 0.1, however, the weight ratio may be 1 to 1. The quantity of use of the powdery activated carbon P is 0.05 to 0.3 g per flow rate of 1 m³/min. The powdery activated carbon P is supplied through an openable and closeable supply port 16 shown in Fig. 6.

Further, a little amount of the anti-bacterium material AB in the powdery state is mixed into the powdery activated carbon P by 0.1-10 %. Silver-loaded zeolite is used as the anti-bacterium material AB. Zeolite itself has a deodorizing function by nature. Further, sterilizing function is exerted by silver coating. The anti-bacterium material AB contained in the powdery activated carbon P like this contains a sterilizer. Besides, particular kinds of seashell powder like a common scallop powder, a titanium powder, etc. exerting the sterilizing function can also be used.

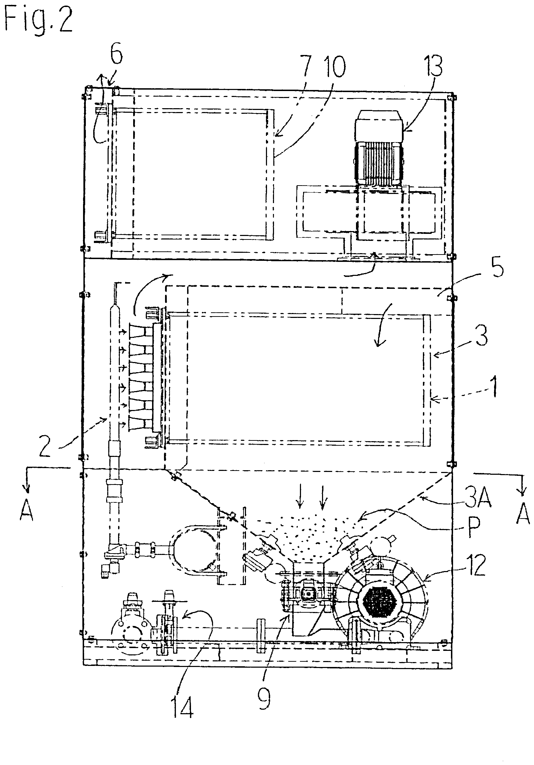

The flow rate of the blower 13 is 30 m³/min, the static pressure thereof is -3.44 kPa, and the output of a drive motor is 4.5 KW. The compressed air of the pulse jet mechanism 2 is stored in a tank 2 by a vane-type compressor 14 for use. The structure of the pulse jet having a number of blowing nozzles (6 nozzles are shown in Fig. 2) is used widely in a dust collector and is publicly known. Therefore, detailed explanation is omitted here. In the meantime, in the case of an ordinary dust collector, the pulse jet mechanism 2 is operated usually while blocking a suction negative pressure by a damper. However, in this embodiment, in order to simplify the structure by eliminating installation of the damper etc., the pulse jet mechanism 2 is operated while the dust collector is operated. The powdery activated carbon P is sufficiently dusted off unlike general fine dust.

The cyclical supply section 4 comprises a rotary valve 9 discharging the powdery activated carbon P dusted off and dropped on a bottom of a hopper 3A of the deodorizing section 3 by a fixed quantity, a reflux pipe 11 refluxing the powdery activated carbon P from the rotary valve 9 to the deodorizing section 3, and a blower 12 for refluxing the powdery activated carbon P to the deodorizing section 3 through the reflux pipe 11 by pneumatic transmission. The flow rate of the blower 12 which is so-called a ring blower is 2.5 m3/min, the static pressure thereof is -4.90 kPa, and the output of the drive motor thereof is 0.85 KW.

A switchover valve 15 is arranged on a discharge side of the rotary valve 9. When wholly or partially replacing the above-mentioned powdery activated carbon P, which has been used for a long period, with new powdery activated carbon P, the switchover valve 15 is operated to discharge the used powdery activated carbon P. Upon usual operation, the powdery activated carbon P is refluxed to the reflux pipe 11.

In the bag-filter type deodorizing apparatus which is structured as mentioned above, the powdery activated carbon P is supplied by a fixed quantity into the hopper 3A of the deodorizing section 3 from an openable and closeable supply port 16 shown in Fig. 6. Then, the blower 13 and the blower 12 of the cyclical supply section 4 are driven.

When the blower 12 of the cyclical supply section 4 is driven, the powdery activated carbon P supplied to the bottom of the hopper 3A of the deodorizing section 3 is discharged near the intake port 5 of the deodorizing section 3 via the rotary valve 9 and through the reflux pipe 11 by pneumatic transmission. The powdery activated carbon P reaches the inside of the deodorizing section 3 while being in contact with the ambient air to be deodorized. The ambient air to be deodorized is filtered by the powdery activated carbon P and is deodorized at the same time.

The deodorized ambient air is taken into the blower 13, and discharged to the auxiliary filtering section 7, where the deodorized ambient air is filtered again by the bag filter section 10. After that the deodorized ambient air is discharged to the outside of the deodorizing apparatus through the discharge port 6.

Therefore, even when the powdery activated carbon P passes through the bag filter section 1 because of breakage of the bag filter section 1, the bag filter 10 of the auxiliary filtering section 7 prevents the powdery activated carbon P from being directly discharged to the outside.

The blower 13 discharges the ambient air to the auxiliary filtering section 7 without discharging the same directly to the outside, and then is discharged to the outside through filtering by the bag filter section 10 arranged in the auxiliary filtering section 7. Therefore, the auxiliary filtering section 7 functions as a buffering section to buffer generation of noise, so that the noise reduction effect can be attained. According to experiments, the noise level is around 80 dB when the auxiliary filtering section 7 is not arranged, but it is reduced to around 55 dB by arranging the same.

The bag-filter type deodorizing apparatus of this invention is installed in a place like a factory for processing raw food like odor-generating sea food, and a hospital generating less noise by nature, or in an odor-generating foundry, where deodorizing function can be locally exerted. Thus, the bag-filter type deodorizing apparatus can be applied to various places where odor is generated.

### Description of Code

1: Bag Filter Section
2: Pulse Jet Mechanism
3: Deodorizing Section
4: Cyclical Supply Section
5: Intake Port
6: Discharge Port
7: Auxiliary Filtering Section
10: Bag Filter Section (Auxiliary Filtering Section)
13: Blower
AB: Antibacterial Material
P: Powdery Activated Carbon

## Claims

1. A bag-filter type deodorizing apparatus including a deodorizing section (3) having a similar structure as that of a bag-filter type dust collector provided with a bag filter section (1) and with a pulse jet mechanism dusting off the bag filter section (1), and a cyclical supply section (4) cyclically supplying to the deodorizing section (3) powdery activated carbon (P) serving as a deodorizing powder body for deodorizing the ambient air taken in through an intake port (5) by a blower (13) by using the powdery activated carbon (P), and for discharging the air through a discharge port (6), the bag-filter type deodorizing apparatus comprising:
an auxiliary filtering section (7) provided with a bag filter section (10) and fitted to the deodorizing section (3);
wherein the blower (13) is arranged between the deodorizing section (3) and the auxiliary filtering section (7), and the ambient air deodorized by the deodorizing section (3) is sent into the auxiliary filtering section (7) and is discharged to the outside through the auxiliary filtering section (7).

2. The bag-filter type deodorizing apparatus as claimed in claim 1, wherein the blower (13) is arranged above the deodorizing section (3) and the auxiliary filtering section (7) is arranged above the deodorizing section (3) and beside the blower (13), whereby sending the deodorized ambient air taken from the lower part sideways, and discharging the same to the outside through the bag-filter section (10) of the auxiliary filtering section (7).

3. The bag-filter type deodorizing apparatus as claimed in claim 1 or 2, wherein the bag filter section (1) of the deodorizing section (3) and the bag-filter section (10) of the auxiliary filtering section (7) are arranged in a manner that the longitudinal direction of the bag-shaped body of the bag filter is oriented laterally.

4. The bag-filter type deodorizing apparatus as claimed in one of the claims 1 to 3 further comprising quick lime in the powdery state which is mixed into the powdery activated carbon (P).

5. The bag-filter type deodorizing apparatus as claimed in one of the claims 1 to 4 further comprising an antibacterial material (AB) in the powdery state which is mixed into the powdery activated carbon (P).
